# EUROPÄISCHE PATENTSCHRIFT

(11) **EP 0 592 794 B1**
(45) Veröffentlichungstag und Bekanntmachung des Hinweises auf die Patenterteilung: **18.06.1997**
(21) Anmeldenummer: 93113662.6
(22) Anmeldetag: 26.08.1993
(51) Int. Cl.: F21V 9/16

(54) **Vorrichtung zur Erzeugung und Emission Elektromagnetischer Strahlung**
Apparatus for generating and emitting electromagnetic radiation
Dispositif pour engendrer et émettre un rayonnement électro-magnétique

(30) Priorität: 26.08.1992 DE 4228311
(43) Veröffentlichungstag der Anmeldung: 20.04.1994
(73) Patentinhaber: IMAB-STIFTUNG, FL-9496 Balzers (LI)
(72) Erfinder: Brück, Gernot K., D 50858 Köln (DE)
(74) Vertreter: Lippert, Hans-Joachim, Dipl.-Ing.

(56) Entgegenhaltungen:
- FR-A- 2 634 539
- GB-A- 540 898
- US-A- 5 211 467

## Beschreibung

Die Erfindung betrifft eine Vorrichtung zur Erzeugung und Emission elektromagnetischer Strahlung im UV-Bereich, bei der lumineszierende Substanzen wie Phosphore durch eine Strahlenquelle, zur Lichtemission angeregt werden und bei der die lumineszierenden Substanzen in oder auf einem Träger angeordnet sind, der mit der Strahlenquelle verbindbar ist und der die Strahlenquelle zumindest teilweise umgibt.

Eine gattungsgemäße bekannte Vorrichtung besteht beispielsweise aus einer Niederdruckquecksilber-Dampflampe, bei der sich im Inneren einer Röhre aus Glas oder Quarzglas eine geringe Menge Quecksilber in einem Gasgemisch geringen Druckes befindet. Auf der Innenseite des Glasrohres sind bestimmte Phosphorgemische aufgetragen.

Nachdem das Gasgemisch im Inneren der Röhre durch einen Hochspannungsimpuls gezündet wurde, wird das angeregte Plasma durch die Betriebsspannung am Brennen gehalten. Für die durch den Elektronenfluß angeregte Strahlung ist fast ausschließlich das in der Röhre befindliche Quecksilber verantwortlich. Die Hauptstrahlung erfolgt bei der Wellenlänge 253 nm, also dem kurzwelligen UV-Licht.

Diese Strahlung kann aber das normale Glasrohr nicht passieren, da das verwendete Glas für diese Wellenlänge nicht transparent ist. Die auf der Röhreninnenseite befindlichen Phosphore werden jedoch durch diese energiereiche Strahlung angeregt und emittieren weniger energiereiche Strahlung im langwelligen UV- und/oder im sichtbaren Bereich.

Die Lebensdauer der Lampe hängt im wesentlichen von den Phosphoren ab, da sich die elektrischen Daten und damit die innere Strahlung der Röhre nur unwesentlich über die Zeit ändern.

Mit modernen Phosphoren für Strahlung im sichtbaren Bereich wird beispielsweise eine Lebensdauer von ca. 8000 bis 10000 Stunden erreicht.

Bei UV-Röhren, die z.B. bei Bräunungsgeräten, in Gewächshäusern, zur Aquarienbeleuchtung und dgl. eingesetzt werden, ist die Lebensdauer jedoch wesentlich geringer. Aufgrund des geringen Energieabstandes zwischen der Anregungsstrahlung und dem emittierten Licht ist es wesentlich schwieriger, langlebige Phosphore bereitzustellen. Bei derartigen Röhren fällt die Strahlungsleistung schon in den ersten 50 Betriebsstunden bis zu 10 % ab. In der Regel müssen z.B. in industriell gefertigten Bräunungsgeräten die mit speziellen Phosphoren beschichteten Röhren schon nach 300 bis 400 Stunden gewechselt werden, obwohl die Lebensdauer der Lampe wesentlich höher sein könnte. Die Strahlungsintensitätseinbuße durch die verwendeten kurzlebigen Phosphore führt auch zu Problemen hinsichtlich der üblicherweise fest eingestellten Taktzeiten der Bräunungsgeräte.

Ein Austausch der auf der Innenfläche der Röhren angeordneten Phosphore ist aber fertigungstechnisch nach dem bekannten Stand der Technik zu aufwendig, so daß die an sich noch intakten Röhren relativ rasch ausgetauscht werden müssen.

Eine gattungsgemäße Vorrichtung wird in der GB-A-540 898 beschrieben, bei der eine elektrische Gasentladungsröhre in einem mit einem stabilen Schirm versehenem Gehäuse angeordnet ist. Der Schirm ist aus einem für UV-Strahlung transparenten Material gefertigt, in welches lumineszierende Substanzen eingelagert sind. Aufgrund der Anordnung der lumineszierenden Substanzen im Leuchtschirm brauchen diese bei einem Defekt der Lampe nicht ebenfalls ausgetauscht zu werden und sind des weiteren von einem direkten Kontakt mit dem Plasma der Gasentladungsröhre geschützt.

Des weiteren ist aus der FR-A-2 634 539 eine Beleuchtungseinrichtung zur Emission von farbigem Licht bekannt, die vorwiegend als Lichtsignal einsetzbar ist. Um bei gleicher Beleuchtungsstärke für farbiges Licht die Beleuchungseinrichtung mit geringerer Leistung betreiben zu können, wird vorgeschlagen, vor der Lichtquelle eine mit einem fluoreszierenden Stoff versehene Folie anzuordnen, wobei vor dieser Folie des weiteren eine farbige, beispielsweise rote Folie angeordnet wird. Die Wellenlänge des von dem fluoreszierenden Stoff emittierten Lichtes entspricht der Farbe der Farbfolie.

Der Erfindung liegt die Aufgabe zugrunde, eine Vorrichtung zur Erzeugung und Emission elektromagnetischer Strahlung im UV-Bereich zu schaffen, die bei möglichst langer Lebensdauer einen möglichst hohen Nutzlichtanteil der emittierten Strahlung im UV-A- und UV-B-Bereich aufweist und die konstruktiv einfach ausgeführt und vielseitig einsetzbar ist.

Diese Aufgabe wird erfindungsgemäß dadurch gelöst, daß der Träger als Kunststoffolie mit zwei oder mehreren Folienschichten ausgebildet ist, von welchen mindestens eine als lumineszierende Substanzen UV-A- und/oder UV-B-Licht emittierende Phosphore enthält und im wesentlichen UV-transparent ist und mindestens eine weitere von der Strahlenquelle weiter beabstandete Folienschicht im wesentlichen keine Transmission für kurz- und/oder langwelligere Strahlung aufweist.

Durch die erfindungsgemäße Kunststoffolie ist eine Lichtquelle geschaffen, die einen hohen Nutzlichtanteil der emittierten Strahlung im UV-A- und/oder im UV-B-Bereich und im wesentlichen keine Anteile in dem an den Nutzlichtbereich angrenzenden Spektralbereich aufweist und die zugleich eine konstruktiv und in der Handhabung besonders einfache und langlebige Ausgestaltung einer Strahlenquelle ermöglicht.

Beim Nachlassen der Strahlungsintensität durch die Kurzlebigkeit der verwendeten Phosphore kann die Folie durch eine neue ersetzt werden, wobei die entsprechende Kunststoffolie z. B. bei UV-Röhren auf der Außenseite angeordnet sein kann und das für die Glasröhre verwendete Glasmaterial beispielsweise bei Quecksilberdampflampen das anregende UV-C-Licht mit einer Wellenlänge von 253 nm ohne bedeutende Verluste passieren läßt. Derartige Gläser werden zum Beispiel bei Entkeimungslampen eingesetzt, die fast ausschließlich Strahlung der Wellenlänge 253 nm emittieren.

Die erfindungsgemäße Kunststoffolie kann dabei hinsichtlich ihrer Lichtdurchlässigkeit so aufgebaut sein, daß die energiereiche Strahlung von 253 nm nicht unkontrolliert nach außen emittiert wird.

Die bei der erfindungsgemäßen Vorrichtung vorzugsweise verwendeten UV-durchlässigen Folien enthalten Phosphore, die auf die Folie aufgetragen oder darin oberflächennah eingearbeitet sind. Darüber hinaus können sich die Phosphore zwischen zwei Folien befinden und von einer Seite her mit UV-C-Licht bestrahlt werden. Handelt es sich bei den Folienschichten um solche, die UV-C-transparent sind, so handelt es sich um Anregungsfolienschichten. Wird jedoch von der Folienschicht UV-C-Licht absorbiert und nur UV-B-Licht in gewissen Grenzen und UV-A-Licht voll transmittiert, so handelt es sich um eine Emissionsfolienschicht. Wird eine Emissionsfolie aus zwei miteinander verschweißten Folien gebildet, so muß eine der Folien UV-C-transparent sein. Bei den Emissionsfolien ist die Anregungsseite die Seite, auf der sich die Phosphore befinden bzw. die Folie, die UV-C-durchlässig ist. Die Trägerfolie selbst weist keine Transmission für UV-C-Licht auf, wohl aber für UV-B- und UV-A-Licht. Durch die Auswahl der Trägerfolie und deren Dicke können auch die Emissionscharakteristika beeinflußt werden. Der Energieshift von der kurzwelligen Anregungsstrahlung zur langwelligeren Nutzstrahlung findet dann in der Folie statt.

Neben der Anregung durch UV-C-Strahlung sind auch Anregungen der Phosphore durch radioaktive Substanzen, insbesondere reine Alpha-Strahler, durch hochfrequente elektromagnetische Felder, durch Spannungspotentiale usw. anwendbar.

Die verwendete Folie besteht vorzugsweise aus Fluorpolymeren, da dieser Kunststoff UV-beständig bzw. -durchlässig ist.

Eine erfindungsgemäße Vorrichtung besteht z.B. aus einer einfachen Niederdruckquecksilber-Dampflampe ohne jede Phosphorbeschichtung mit einer Lebensdauer von vielen tausend Stunden, die mit einem dünnen Schlauch aus Phosphorfolie mit den gewünschten Phosphoren überzogen wird, wobei die Anregungsseite zur Röhre gewandt ist. Darüber hinaus kann, z.B. bei einem Bräunungsgerät wie einer Sonnenbank, eine Vielzahl von UV-C-Röhren in einem Gehäuse untergebracht sein, wobei die Röhren ausschließlich eine intensive Strahlung im UV-C-Bereich emittieren und die Röhrenleistung beispielsweise zwischen 100 und 160 Watt liegt.

Das Gehäuse weist auf einer Seite eine Öffnung auf, die sich über die gesamte Fläche hinzieht. Zur Verstärkung der Strahlung kann das gesamte Gehäuseinnere verspiegelt sein. Die Öffnung wird von einer Folie abgeschlossen, die die entsprechenden Phosphore enthält und so angeordnet ist, daß die Anregungsseite den Röhren zugewandt ist. Auf der Rückseite wird dann das phosphor- und folienspezifische Licht ausgestrahlt. Hierdurch entsteht eine kräftige Nutzstrahlung auf einer großen Fläche, wie sie in den Bräunungsgeräten erwünscht ist.

Bei einer derartigen Anordnung lassen sich die UV-C-Röhren auch durch andere Quecksilberdampflampen ersetzen, möglich sind, wie bereits erwähnt, jedoch auch völlig andere Anregungssysteme.

Bei einer vorteilhaften Weiterbildung der Erfindung können die Phosphorfolien in einem Rahmen schnell getauscht werden oder mittels einer Rolle ein neues Stück eingezogen werden. Auch sind mehrere Folien einsetzbar, wobei die näher zu den UV-C-Röhren liegenden Folien voll UV-C-transparent sind.

Bei einer entsprechenden Dicke der UV-durchlässigen Folien ist es auch möglich, diese gleichzeitig als Unterlage bzw. Liege für den Benutzer eines Bräunungsgerätes einzusetzen.

Insbesondere bei Quecksilberdampf-Hochdruckbrennern können die Phosphorfolien als aktive Filter eingesetzt werden. Durch entsprechende Einfärbung der Folien kann eine Blockung des sichtbaren Lichtes erreicht werden. Die Transmissionskurve zum kurzwelligen UV-Licht kann durch die richtige Auswahl des Folienmaterials eingestellt werden. Der Einsatz als aktive Lichtfilter hat dabei den Vorteil, daß im Gegensatz zu den bisher verwendeten üblichen Filtern, bei denen nur eine ausgewählte Wellenlänge den Filter passieren kann, der ganze Bereich der kurzwelligen Strahlung nicht einfach verschluckt wird, sondern über den Energieshift der Phosphore in zusätzlich nutzbares Licht z.B. im Bereich der UV-B- und UV-A-Strahlung umgewandelt werden kann.

Die erfindungsgemäße Vorrichtung ist überall da einsetzbar, wo die Lebensdauer durch die Kurzlebigkeit der verwendeten fotolumineszierenden Substanzen begrenzt wird oder es zu Veränderungen der gewünschten Strahlungsart bzw. Strahlungszusammensetzung kommt, also z.B. bei Bräunungsgeräten Lampen für die Beleuchtung von Tieren oder Pflanzen oder sonstigen Spezialröhren.

Die Erfindung ist in der Zeichnung beispielsweise veranschaulicht und wird im nachfolgenden anhand der Zeichnung im einzelnen erläutert. Es zeigen:
- Fig. 1 - 3: unterschiedlich ausgebildete Phosphorfolien in einer Schnittdarstellung,
- Fig. 4: ein Gehäuse mit mehreren UV-C-Lampen in einer Draufsicht,
- Fig. 5 und 6: eine Seitenansicht bzw. einen Schnitt durch eine UV-C-Lampe,
- Fig. 7: einen Schnitt durch eine Sonnenbank gem. Fig. 4 mit einem Folienrahmen,
- Fig. 8: einen Schnitt durch eine Sonnenbank gem. Fig. 4 mit einer auf- bzw. abrollbaren Phosphorfolie und
- Fig. 9: in einer Schemadarstellung die Anordnung bei einer Verwendung der Phosphorfolie als Aktivfilter.

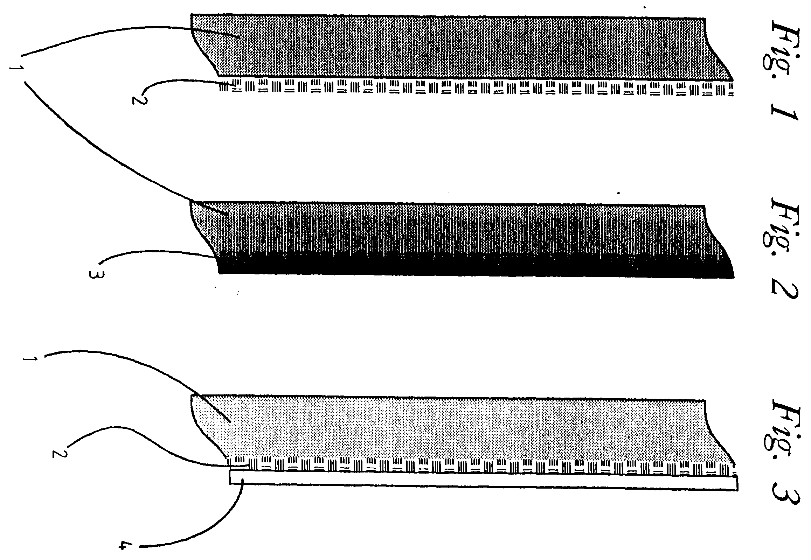

In den Figuren 1 bis 3 werden Phosphorfolien gezeigt, die für eine erfindungsgemäße Vorrichtung eingesetzt werden können. Gemäß Fig. 1 besteht die Phosphorfolie aus einer Folie 1, auf die eine Phosphorschicht 2 aufgetragen ist. Eine andere Möglichkeit ist das Einarbeiten der Phosphore in die Oberfläche 3 der Folie 1. Zum Schutz der Phosphorschicht 2 kann diese mit einer weiteren dünnen Schutzfolie 4 abgedeckt sein (Fig. 3).

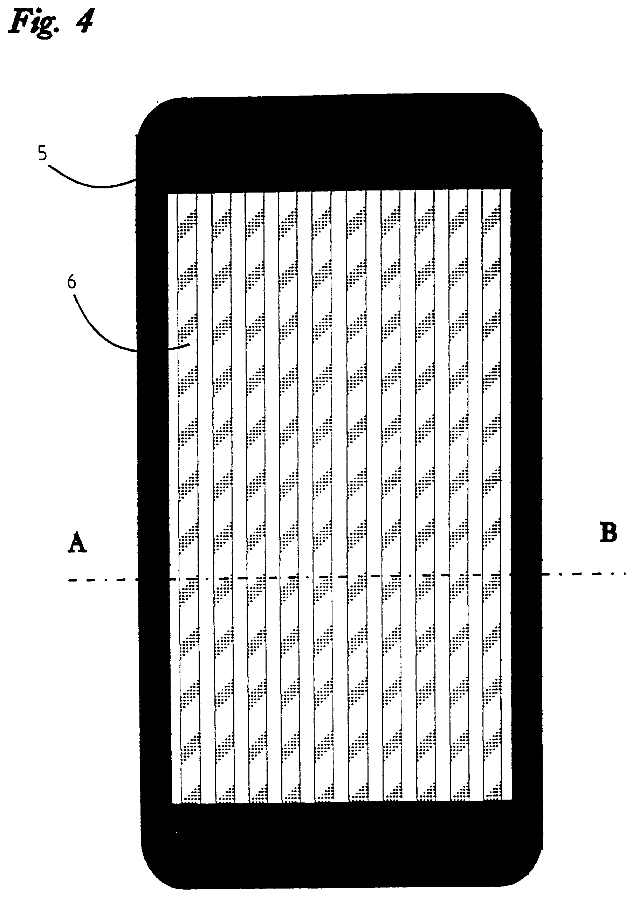

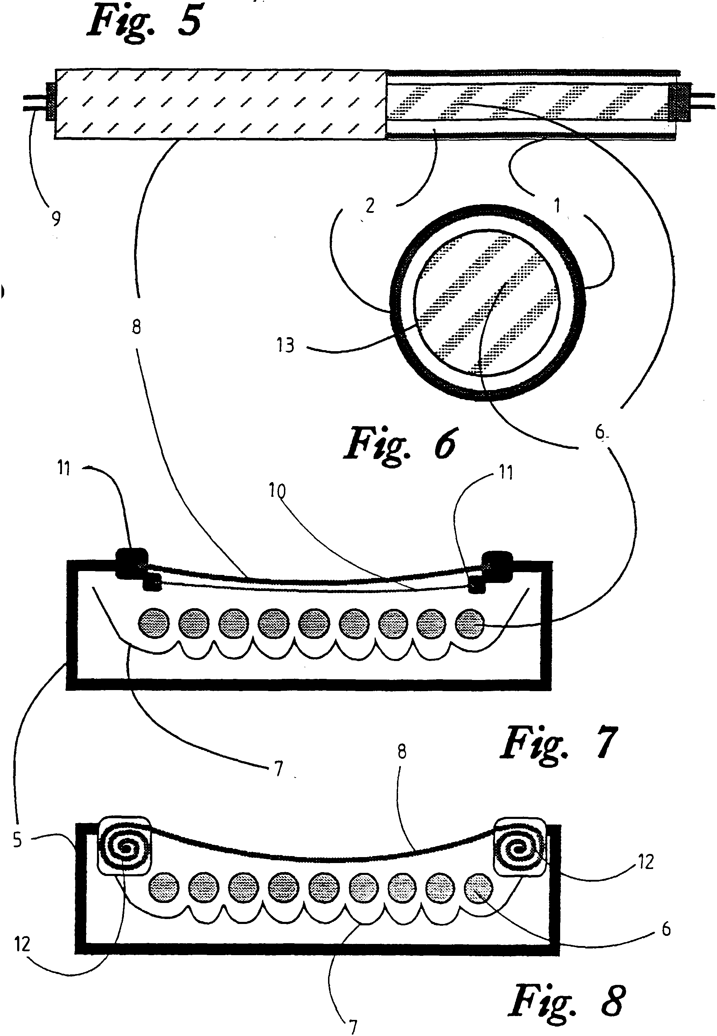

In Fig. 4 wird eine mögliche Ausführungsform dargestellt, wobei sich in einem Gehäuse 5 mehrere UV-C-Lampen 6 befinden. Die mögliche Anordnung der UV-C-Röhren 6 im Gehäuse 5 und die Reflektorspiegelfläche 7 ist in der Schnittdarstellung durch die Ebene A-B in den Fig. 7 und 8 dargestellt. Die Gehäuseöffnung wird abgeschlossen durch eine Phosphorfolie 8, die sich in einem Rahmen 9 befindet.

Unterhalb der Phosphorfolie 8, die hier auch als Emissionsfolie bezeichnet wird, befindet sich eine weitere Phosphorfolie 10, die voll UV-C-durchlässig ist und auch als Anregungsfolie bezeichnet wird. Auch diese befindet sich in einem Rahmen 11.

Eine weitere Möglichkeit zur Anbringung der Phosphorfolie 8 wird in Fig. 8 dargestellt, durch eine Abroll- bzw. Aufrollvorrichtung 12 kann die verbrauchte Phosphorfolie aufgerollt werden, wodurch frische Phosphorfolie aus der Abrollvorrichtung 12 gezogen wird.

Die Ausrüstung einer einzelnen UV-C-Röhre wird in den Fig. 5 und 6 gezeigt. Ein Schlauch aus Phosphorfolie 8 wird über die UV-C-Röhre 6 gezogen, wobei in der Schnittfigur 6 die Glaswand 13 der UV-C-Röhre zu erkennen ist, sowie die Anordnung der Phosphorschicht 2 auf der Folie 1, die zur Röhre 6 gewandt ist.

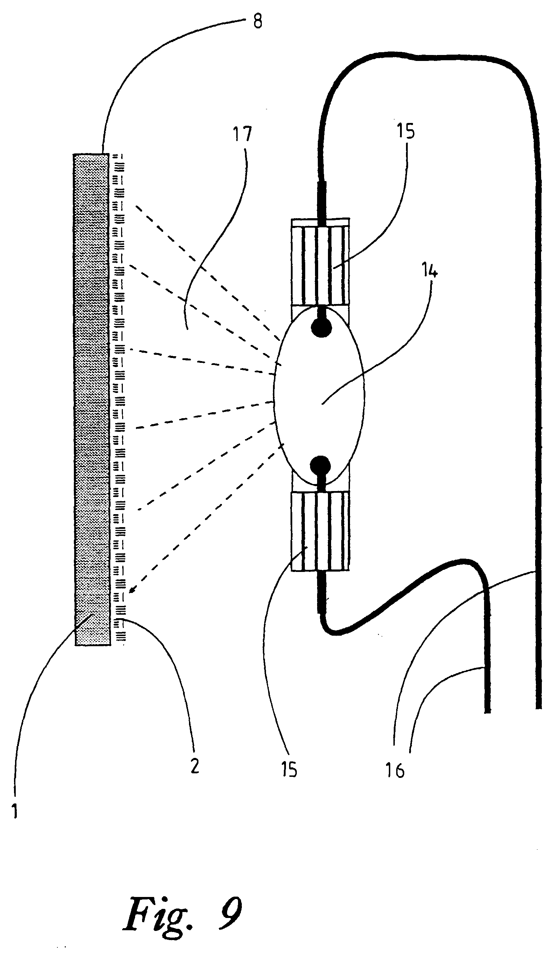

Gemäß Fig. 9 wird die Phosphorfolie als Aktivfilter eingesetzt. Aus der Quecksilberdampf-Hochdrucklampe 14 mit den Elektrodenquetschungen 15 und den Anschlußdrähten 16 tritt die breidbandige Strahlung 17 aus und trifft auf die Phosphorfolie 8, deren Folienmaterial 1 UV-durchlässig ist, aber so eingefärbt ist, daß das sichtbare Licht absorbiert wird. Das UV-A- und Teile des UV-B-Lichtes können die Folie passieren. Das energiereichere UV-B- und das gesamte UV-C-Licht werden in der Phosphorschicht 2 aufgefangen und regen den Phosphor an, seine spezifische Strahlung zu emittieren.

### Bezugsseichenliste

- 1: UV-durchlässige Folie
- 2: Phosphorschicht
- 3: Oberfläche
- 4: Schutzfolie
- 5: Gehäuse
- 6: UV-C-Röhre
- 7: Reflektorspiegelfläche
- 8: Phosphorfolie
- 9: Rahmen
- 10: Phosphorfolie
- 11: Rahmen
- 12: Aufrollvorrichtung
- 13: Glaswand
- 14: Quecksilberdampf-Hochdrucklampe
- 15: Elektrodenquetschungen
- 16: Anschlußdraht
- 17: Strahlung

## Patentansprüche

1. Vorrichtung zur Erzeugung und Emission elektromagnetischer Strahlung im UV-Bereich, bei der lumineszierende Substanzen wie Phosphore durch eine Strahlenquelle, zur Lichtemission angeregt werden und bei der die lumineszierenden Substanzen in oder auf einem Träger angeordnet sind, der mit der Strahlenquelle verbindbar ist und der die Strahlenquelle zumindest teilweise umgibt, **dadurch gekennzeichnet**, daß der Träger als Kunststoffolie (1) mit zwei oder mehreren Folienschichten ausgebildet ist, von welchen mindestens eine als lumineszierende Substanzen UV-A- und/ oder UV-B-Licht emittierende Phosphore enthält und im wesentlichen UV-transparent ist, und daß mindestens eine weitere, von der Strahlenquelle weiter beabstandete Folienschicht vorgesehen ist, die im wesentlichen keine Transmission für kurz- und/oder langwelligere Strahlung aufweist.

2. Vorrichtung nach Anspruch 1, **dadurch gekennzeichnet**, daß die Kunststoffolie aus Fluorpolymeren besteht.

3. Vorrichtung nach Anspruch 1 oder 2, **dadurch gekennzeichnet**, daß die Kunststoffolie (1) als Verbundfolie mit zwei oder mehreren Folienschichten ausgebildet ist, von welchen mindestens eine die lumineszierenden Substanzen enthält.

4. Vorrichtung nach Anspruch 1 bis 3, **dadurch gekennzeichnet**, daß die lumineszierenden Substanzen durch UV-C-Strahlung zur Lichtemission anregbar sind.

5. Vorrichtung nach einem der Ansprüche 1 bis 4, **dadurch gekennzeichnet**, daß die Strahlenquelle aus einer oder mehreren UV-C-Röhren (6), insbesondere Quecksilberdampfröhren besteht, die jeweils von einer Kunststoffolie (1) mit lumineszierenden Substanzen schlauchartig überzogen oder deren gemeinsames Gehäuse (5) eine Öffnung aufweist, die mit einer derartigen Folie (1) abgedeckt ist.

6. Vorrichtung nach einem der Ansprüche 1 bis 5, **dadurch gekennzeichnet**, daß die Kunststoffolie (1) auswechselbar angeordnet ist.

7. Vorrichtung nach einem der Ansprüche 1 bis 6, **dadurch gekennzeichnet**, daß die Kunststoffolie (1) als Unterlage für den Benutzer eines Bräunungsgerätes wie einer Sonnenbank ausgebildet ist.

8. Vorrichtung nach einem der Ansprüche 1 bis 7, **dadurch gekennzeichnet**, daß die Kunststoffolie (1) als aktiver Lichtfilter mit oder ohne Einfärbung des Folienmaterials einsetzbar ist.

9. Vorrichtung nach einem der Ansprüche 1 bis 8, **dadurch gekennzeichnet**, daß ein Meßgerät zur Erfassung und/oder Analyse elektromagnetischer Strahlung vorgesehen ist, das bei Abweichungen von der gewünschten, durch die Anregung der verwendeten lumineszierender Substanzen erzeugten Nutzstrahlung ein Alarmsignal erzeugt oder die Strahlenquelle ausschaltet.

## Claims

1. Device for the production and emission of electromagnetic radiation in the UV range, in which luminescent substances such as phosphorus are excited to emit light by a radiation source, and in which the luminescent substances are disposed in or on a support which can be connected to the radiation source and which at least partially surrounds the radiation source, characterized in that the support is formed as plastics sheeting (1) with two or a plurality of layers, of which at least one contains UV-A- and/or UV-B-light-emitting phosphorus as luminescent substances and is substantially UV-transparent, and in that at least one further layer is provided, disposed further from the radiation source and showing substantially no transmission for short- and/or long-wave radiation.

2. Device according to Claim 1, characterized in that the plastics sheeting is composed of fluoropolymers.

3. Device according to Claim 1 or Claim 2, characterized in that the plastics sheeting (1) is formed as compound sheeting with two or a plurality of layers, of which at least one contains the luminescent substances.

4. Device according to Claims 1 to 3, characterized in that the luminescent substances can be excited to emit light by UV-C-radiation.

5. Device according to any one of Claims 1 to 4, characterized in that the radiation source consists of one or a plurality of UV-C-tubes (6), particularly mercury-vapour tubes, which are covered by respective tubes of plastics sheeting (1) with luminescent substances, or of which the common housing (5) has an opening which is covered by a sheeting (1) of this type.

6. Device according to any one of Claims 1 to 5, characterized in that the plastics sheeting (1) is arranged so as to be replaceable.

7. Device according to any one of Claims 1 to 6, characterized in that the plastics sheeting (1) is formed as an underlay for the user of tanning equipment such as a sun bed.

8. Device according to any one of Claims 1 to 7, characterized in that the plastics sheeting (1) is usable as an active light filter with or without tinting of the sheeting material.

9. Device according to any one of Claims 1 to 8, characterized in that a measuring device for the detection and/or analysis of electromagnetic radiation is provided and, upon deviations from the desired useful radiation produced by the excitation of the luminescent substances used, produces an alarm signal or switches off the radiation source.

## Revendications

1. Dispositif pour engendrer et émettre un rayonnement électromagnétique dans le domaine de l'UV, dans lequel des substances luminescentes telles que des phosphores sont excitées par une source de rayonnement en vue de l'émission de lumière, et dans laquel les substances luminescentes sont disposées dans ou sur un support qui peut être fixé à la source de rayonnement et qui entoure au moins partiellement la source de rayonnement, caractérisé en ce que le support est réalisé sous forme de feuille de matière plastique (1) à deux ou plusieurs couches de feuille dont l'une au moins contient, en tant que substances luminescentes, des phosphores émettant de la lumière UV-A et/ou UV-B et est essentiellement transparente à l'UV, et en ce qu'il est prévu au moins une autre couche de feuille, plus éloignée de la source de rayonnement, qui ne transmet pratiquement pas le rayonnement à plus courte et/ou à plus grande longueur d'onde.

2. Dispositif selon la revendication 1, caractérisé en ce que la feuille de matière plastique est faite de polymère fluoré.

3. Dispositif selon la revendication 1 ou 2, caractérisé en ce que la feuille de matière plastique (1) est réalisée sous forme de feuille composée à deux ou plusieurs couches de feuille dont l'une au moins contient les substances luminescentes.

4. Dispositif selon l'une quelconque des revendications 1 à 3, caractérisé en ce que les substances luminescentes sont excitables par le rayonnement UV-C en vue de l'émission de lumière.

5. Dispositif selon l'une quelconque des revendications 1 à 4, caractérisé en ce que la source de rayonnement est constituée par un ou plusieurs tubes émetteurs d'UV-C (6), en particulier un ou plusieurs tubes à vapeur de mercure, qui sont revêtus chacun, à la manière d'une gaine, d'une feuille de matière plastique (1) contenant des substances luminescentes, ou dont le boîtier commun (5) comporte une ouverture qui est masquée par une telle feuille (1).

6. Dispositif selon l'une quelconque des revendications 1 à 5, caractérisé en ce que la feuille de matière plastique (1) est disposée de manière à être interchangeable.

7. Dispositif selon l'une quelconque des revendications 1 à 6, caractérisé en ce que la feuille de matière plastique (1) est réalisée sous forme de surface de repos pour l'utilisateur d'un appareil de bronzage tel qu'un banc de bronzage.

8. Dispositif selon l'une quelconque des revendications 1 à 7, caractérisé en ce que la feuille de matière plastique (1) est utilisable comme filtre de lumière actif, avec ou sans coloration de la matière de la feuille.

9. Dispositif selon l'une quelconque des revendications 1 à 8, caractérisé en ce qu'il est prévu, pour la détection et/ou l'analyse du rayonnement électromagnétique, un appareil de mesure qui émet un signal d'alarme ou coupe la source de rayonnement en cas d'écarts par rapport au rayonnement utile désiré, produit par excitation des substances luminescentes utilisées.
